# EUROPEAN PATENT APPLICATION

(11) **EP 3 689 843 A1**
(43) Date of publication of application: **05.08.2020**
(21) Application number: 19154999.7
(22) Date of filing: 01.02.2019
(51) Int. Cl.: C07C 2/00, C07C 2/76, C10G 57/00

(54) **A METHOD FOR PRODUCING AN AROMATIC HYDROCARBON OR A MIXTURE OF AROMATIC HYDROCARBONS FROM A LOW MOLECULAR HYDROCARBON OR A MIXTURE OF LOW MOLECULAR HYDROCARBONS**

(71) Applicant: BASF SE, 67056 Ludwigshafen am Rhein (DE)
(72) Inventor: TOMPERS, Rolf, 67056 Ludwigshafen (DE); KUHN, Jelan, 200233 Shanghai (CN); CZAJKA, Pawel, 67056 Ludwigshafen (DE); REISER, Michael, 67056 Ludwigshafen (DE); FEYEN, Mathias, 67056 Ludwigshafen (DE)
(74) Representative: BASF IP Association

(57) **Abstract**

A method for producing an aromatic hydrocarbon or a mixture of aromatic hydrocarbons from a low molecular hydrocarbon or a mixture of low molecular hydrocarbons, wherein, in a first step, a low molecular hydrocarbon or a mixture of low molecular hydrocarbons is converted to a mixture of aromatic hydrocarbons by way of a catalytic dehydroaromatization reaction with hydrogen as a byproduct, separating the mixture of aromatic hydrocarbons from hydrogen and nonaromatic hydrocarbons in a second step, and, in a third step, feeding the mixture of aromatic hydrocarbons into an aromatic hydrocarbons separation unit, e.g. into the aromatic hydrocarbons separation unit of a catalytic reforming plant, in particular into the aromatic hydrocarbons separation unit of a hydrocarbon steamcracker plant.

## Description

The present invention relates to a method for producing an aromatic hydrocarbon or a mixture of aromatic hydrocarbons from a low molecular hydrocarbon or a mixture of low molecular hydrocarbons.
In a specific embodiment the invention relates to a method for producing an aromatic hydrocarbon or a mixture of aromatic hydrocarbons, such as benzene, toluene and/or xylenes (benzene and toluene also abbreviated as BT; benzene, toluene and xylenes also abbreviated as BTX) by integration of an aromatic hydrocarbon(s) producing unit into a steamcracker plant, which includes an aromatic hydrocarbons separation unit.
In a further specific embodiment, the invention relates to a method for producing an aromatic hydrocarbon or a mixture of aromatic hydrocarbons, such as benzene, toluene and/or xylenes by integration of an aromatic hydrocarbon(s) producing unit into a catalytic reforming plant, which includes an aromatic hydrocarbons separation unit.

Processes to produce aromatic hydrocarbons - e.g. esp. benzene, toluene, xylenes - from a stream containing those aromatic hydrocarbons being a product of steam cracking of hydrocarbons are described in Ullmann's Encyclopedia of Industrial Chemistry, Benzene, Vol. 5, pages 244 - 260, 2012 Wiley-VCH Verlag GmbH & Co. KGaA, Weinheim.
Processes of steam cracking of hydrocarbons are described in Ullmann's Encyclopedia of Industrial Chemistry, Ethylene, Vol. 13, pages 465 - 529, 2012 Wiley-VCH Verlag GmbH & Co. KGaA, Weinheim.
Processes for the manufacture of aromatic and non-aromatic hydrocarbons are also summarized in Ullmann's Encyclopedia of Industrial Chemistry, Hydrocarbons, pages 5 - 61, 2014 Wiley-VCH Verlag GmbH & Co. KGaA, Weinheim.
Process Economics Program Report 182B, Aromatic Processes, by Abe Gelbein, (December 2008) reviews the status of the BTX industry and related production and recovery technologies. Process Economics Program Report 29I, Ethylene from Naphtha, by Michael Arne, (December 2014) covers the technology and costs of manufacturing ethylene from naphtha via steam cracking.

US 3,832,413 A (UOP) describes hydrocarbon aromatization to (polynuclear) aromatic hydrocarbons; Rh catalysts on alumina or silica.
US 4,120,910 A (Mobil Oil) describes ethane aromatization using zeolite-based catalysts.

WO 09/076393 A (Shell) teaches the following: splitting of ethane stream; thermal cracking of 1^{st} stream, combining with 2^{nd} stream and introduction into aromatization stage; increase of benzene yield by hydrodealkylation; furthermore, direct ethane aromatization; in addition, conversion of benzene to phenol or styrene.

WO 12/078506 A (Shell) deals with a 2-stage process which allows to produce ethylene and benzene from a single mixed feedstock containing ethane and higher hydrocarbons (basically propane); aromatization of propane in 1^{st} stage; separation of aromatic hydrocarbons; 1^{st} portion of non-aromatic product stream going to ethane cracker, 2^{nd} portion going to ethane aromatization stage.
US 8,835,706 A (Shell): comparable to the previous process of WO 12/078506 A, aim here is to maximize the conversion of both propane and ethane.
WO 12/078509 A (Shell): comparable to the previous process of US 8,835,706 A, this application describes 1^{st} stage propane aromatization and 2^{nd} stage ethane aromatization.
WO 11/053745 A (Shell): comparable to the previous process of WO 12/078509 A, this application describes 1^{st} stage propane and/or butane aromatization and 2^{nd} stage ethane aromatization.

EP 269 297 B1 (BP) describes dehydrocyclodimerisation (DHCD) of ethane/propane/butane; combination with syngas unit and FT reactor (FT = Fischer Tropsch).

US 170088484 A, US 17052854 A, (both ExxonMobil) address the following: aromatization of bottom streams of separated natural gas - C₁/C₂ to pipeline, C₂₊ to aromatization; modular & transportable system for remote applications; at least 2 stages: 1^{st} for C₃₊, 2nd more severe for C₂.
US 17088486 A (ExxonMobil): comparable to the previous process of US 170088484 A and US 17052854 A, this application is concerned with the use of CO₂ containing hydrocarbon feed; consumption of H₂ by CO₂ methanation improves overall aromatic hydrocarbons yield.

The conversion of ethane to aromatic hydrocarbons using zeolites containing Pt as catalysts is described in the following documents:
US 7,186,871 BB (Sabic): catalyst with Pt being deposited on MFI zeolite.
US 8,772,563 BB (Shell): catalyst containing 0,01 to 0,05 wt.-% Pt and < 1 wt.-% Ga and an aluminosilicate; aluminosilicate having a silica/alumina ratio of 20 to 80 and being selected from the group of ZSM-5, ZSM-11, ZSM-12, ZSM-23, ZSM-35.
US 8,809,608 BB (Shell): catalyst containing 0,005 to 0,1 wt.-% Pt and < 0,2 wt.-% Sn, Pb or Ge and an aluminosilicate; aluminosilicate having a silica/alumina ratio of 20 to 80 and being selected from the group of ZSM-5, ZSM-11, ZSM-12, ZSM-23, ZSM-35.
Further documents in this respect are:
US 4350835 A (Mobil Oil) describes a Ga impregnated zeolite for producing aromatic compounds from gaseous paraffinic hydrocarbon feed containing ethane.
EP 0 107 875 A, EP 0 107 876 A, (both Shell) give attention to crystalline Ga-silicates (Ga incorporated in zeolite lattice).
EP 0 107 877 A (Shell) is concerned with the impregnation of zeolite with Ga; the catalyst needs to be reacted to form coke and regenerated in air to enhance activity and C5+ selectivity.
US 4,642,403 A, EP 0 244 162 A, (both BP) teach a corresponding Ga, Rh/ZSM-5 - catalyst. US 6,784,333 A (Sabic) deals with a Pt deposited on Ge containing zeolite, sulfurization of catalyst.

The purpose of the present invention is to provide an improved method, esp. with regard to chemical efficiency, economic efficiency and/or yield, for producing an aromatic hydrocarbon or a mixture of aromatic hydrocarbons from a low molecular hydrocarbon or a mixture of low molecular hydrocarbons.

Accordingly, a method was found for producing an aromatic hydrocarbon or a mixture of aromatic hydrocarbons from a low molecular hydrocarbon or a mixture of low molecular hydrocarbons, wherein, in a first step, a low molecular hydrocarbon or a mixture of low molecular hydrocarbons is converted to a mixture of aromatic hydrocarbons by way of a catalytic dehydroaromatization reaction with hydrogen as a byproduct, separating the mixture of aromatic hydrocarbons from hydrogen and non-aromatic hydrocarbons in a second step, and, in a third step, feeding the mixture of aromatic hydrocarbons into an aromatic hydrocarbons separation unit, e.g. into the aromatic hydrocarbons separation unit of a catalytic reforming plant, in particular into the aromatic hydrocarbons separation unit of a hydrocarbon steamcracker plant.

In an embodiment, in the third step, the aromatic hydrocarbons separation unit of the hydrocarbon steamcracker plant is a pyrolysis gasoline unit (shortly: pygas unit).

The advantages of the method according to the invention in comparison with the state of the art are especially as follows:
- Lower consumption of hydrogen in the aromatic hydrocarbons separation unit of a catalytic reforming plant or of a hydrocarbon steamcracker plant, especially pygas unit, compared with the state-of-art,
- Lower energy consumption (heating, cooling) in the aromatic hydrocarbons separation unit of a catalytic reforming plant or of a hydrocarbon steamcracker plant, especially pygas unit,
- Lower amounts of off-gases from the aromatic hydrocarbons separation unit of a catalytic reforming plant or of a hydrocarbon steamcracker plant, especially pygas unit,
- Higher amount of desired aromatic hydrocarbon byproducts, especially toluene.
Further advantages are described below.

One reason for the lower energy demand described above is the lower amount (in particular practically none) of C6-C8 non-aromatic hydrocarbons compared to an aromatic hydrocarbons containing fraction from a hydrocarbon steamcracker plant or an aromatic hydrocarbons containing fraction from a catalytic reforming plant (also called reformate); (as also shown below in table 3; see also https://en.wikipedia.org/wiki/BTX_(chemistry), website visited on Nov. 14, 2018).

In particular the aromatic hydrocarbon is benzene, toluene, xylenes (one isomer or mixture of isomers), ethylbenzene, styrene, naphthalene or methylnaphthalene (one isomer or mixture of isomers) and the aromatic hydrocarbons are benzene, toluene, xylenes (one isomer or mixture of isomers), ethylbenzene, styrene, naphthalene and/or methylnaphthalene (one isomer or mixture of isomers).

In particular the low molecular hydrocarbon is a C2-C4-alkane and the molecular hydrocarbons are C2-C4-alkanes. C2-C4-alkanes are ethane, propane, n-butane, iso-butane. A preferred alkane is ethane. A preferred mixture of C2-C4-alkanes is for example a mixture containing
50 to 100 % by weight ethane,
0 to 50 % by weight propane, and
0 to 10 % by weight butanes.

In preferred embodiments, the method according to the invention relates to an integration of a system (also named ethane coupling in further text) producing aromatic hydrocarbons (benzene, toluene, o-xylene, m-xylene, p-xylene, ethylbenzene, naphthalene, methylnaphthalene) from low molecular hydrocarbons (especially C2-C4) via a catalytic dehydroaromatization, with hydrogen as a byproduct of the reaction, with an aromatic hydrocarbons separation system into a system comprising a hydrocarbon steamcracker plant, including an aromatic hydrocarbons separation unit (especially pygas unit) by either mixing the aromatic hydrocarbons containing fraction, esp. pyrolysis gasoline, from the hydrocarbon steamcracker plant with the ethane coupling product or by feeding the aromatic hydrocarbons separation unit, especially pygas unit, with ethane coupling product only. The ethane coupling can optionally have a system to separate the hydrogen and CH4 fraction from the C2-C4 hydrocarbons and optionally a system to separate the hydrogen from CH4.

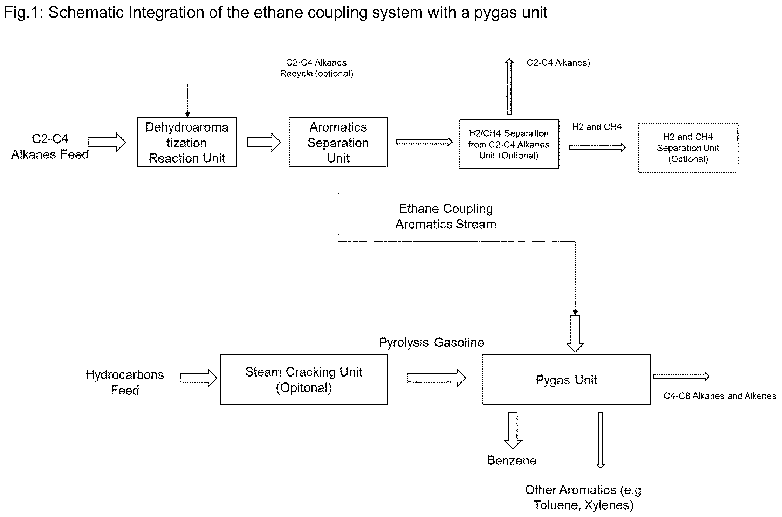

In particular the aromatic hydrocarbons mixture from the ethane coupling system has higher concentration of desired aromatic hydrocarbons, especially benzene and toluene, compared to a standard pygas unit feed (pygas unit feed: pyrolysis gasoline from a hydrocarbon steam cracking unit) leading to lower energy consumption in the pygas unit.
In particular the ethane coupling aromatic hydrocarbons does not contain acetylenes, dienes, styrenes or organic sulfur components which require hydrogenation in the aromatic hydrocarbons separation unit, especially pygas unit. Preferably, the ethane coupling product does not contain significant amounts of C4-C8 non-aromatic alkanes which reduces the energy needed for separation of those components from desired aromatic hydrocarbons.
An exemplary schematic connection of the ethane coupling system with the pygas unit is given in Fig. 1.

In particular after ethane coupling the aromatic hydrocarbons mix is separated from the product mix and fed into the pygas unit of a stream cracker, of which the feed has been changed to lighter feeds, resulting in a lower utilization of the pygas unit. Doing so reduces the idle capacity of the aromatic hydrocarbons section. The remaining product can be fed into the suitable sections of the cracker, these include olefins, hydrogen, and methane. Optionally the olefins can be hydrogenated or directly recycled into the reaction section or fed into the feed stream to the cracker furnaces (reaction section). The produced hydrogen can be used for hydrogenation or purified in existing cracker downstream. Methane / hydrogen could be used as fuel gas. Optionally the gaseous products after aromatic hydrocarbons separation could be used as fuel gas.

In the method according to the invention, in a first step, a low molecular hydrocarbon or a mixture of low molecular hydrocarbons is converted to a mixture of aromatic hydrocarbons by way of a catalytic dehydroaromatization reaction with hydrogen as a byproduct. Other possible byproducts are non-aromatic hydrocarbons, for example olefins, especially alkenes, e.g. ethylene, propene, butenes.
As an example, ethane is converted into mainly mixed aromatic hydrocarbons (benzene, toluene, xylenes and naphthalenes) and olefins and hydrogen as byproducts.
The catalyst in the dehydroaromatization reaction is preferably based on a zeolite loaded with Pt. More preferably, the catalyst based on a ZSM-type zeolite loaded with Pt and Ga or with Pt and Sn. The reaction preferably takes place at 500 - 600 °C and 0 - 10 barg with an ethane conversion of particularly 5 - 60 %, aromatic hydrocarbons selectivity of particularly 70 - 75 %, particularly 20 - 25 % methane and particularly approx. 5 % olefins selectivity.

In the method according to the invention, in a second step, the mixture of aromatic hydrocarbons is separated from hydrogen and non-aromatic hydrocarbons.

In an embodiment, the separation of the mixture of aromatic hydrocarbons from hydrogen and non-aromatic hydrocarbons in the second step is conducted by absorption of the aromatic hydrocarbons in a solvent, as known in the art (e.g.: Ullmann's Encyclopedia of Industrial Chemistry, Benzene, Vol. 5, pages 254 - 260, 2012 Wiley-VCH Verlag GmbH & Co. KGaA, Weinheim; Nexant PERP Benzene/Toluene 2015-7, Section 7 (page 74-88), C. Kothari, H. Lukoma, Dec. 2015).
In particular the non-aromatic hydrocarbons are unconverted low molecular hydrocarbon or unconverted low molecular hydrocarbons, methane, olefins (especially alkenes, e.g. ethylene, propene, butenes).

In a further embodiment, the hydrogen and non-aromatic hydrocarbons separated from the mixture of aromatic hydrocarbons in the second step are fed into a separation unit, resulting in a H2/CH4 - fraction and a low molecular hydrocarbons - fraction (essentially without CH4). In particular, 'essentially' shall mean the fraction contains ≤ 5 % by weight of CH4.
Particularly, the H2/CH4 - fraction is fed into a further separation unit to separate H2 and CH4. Particularly, in addition, the low molecular hydrocarbons - fraction (essentially without CH4) is recycled back to the catalytic dehydroaromatization reaction of the first step or used as feed to an upstream plant, such as a hydrocarbon steamcracker plant or a catalytic reforming plant.

In the method according to the invention, a third step comprises feeding the mixture of aromatic hydrocarbons into an aromatic hydrocarbons separation unit, e.g. into the aromatic hydrocarbons separation unit of a catalytic reforming plant (e.g. Ullmann's Encyclopedia of Industrial Chemistry, Benzene, Vol. 5, pages 244-245 2012 Wiley-VCH Verlag GmbH & Co. KGaA, Weinheim), in particular into the aromatic hydrocarbons separation unit, especially pyrolysis gasoline unit (shortly: pygas unit), of a hydrocarbon steamcracker plant.

In a preferred embodiment, the pygas unit comprises a hydrogenation step for acetylenes and dienes, a C5 splitter [in which a C1 to C5 (C5- fraction) fraction is separated from a C6 to higher-than-C6 fraction (C6+ fraction)], a hydrogenation step for sulfur containing components, a H2S stripping column, a toluene and xylenes separation column and a benzene extractive distillation column. Other preferred embodiments of the pygas unit are those described in Ullmann's Encyclopedia of Industrial Chemistry, Benzene, Vol. 5, pages 237 - 268 (esp. pages 252-259, 2012 Wiley-VCH Verlag GmbH & Co. KGaA, Weinheim, and Process Economics Program Report 182B, Aromatic Processes, by Abe Gelbein, esp. pages 4-7 to 4-13 (112-118), (December 2008).

In particular the aromatic hydrocarbons separation unit of the hydrocarbon steamcracker plant of the third step is in addition fed with an aromatic hydrocarbons containing fraction from the hydrocarbon steamcracker plant.
Preferably, the aromatic hydrocarbons containing fraction from the hydrocarbon steamcracker plant is pyrolysis gasoline (shortly: pygas).
Typically, pyrolysis gasoline is (as a by-product from steam cracking, of e.g. naphta, for olefins production) a mixture comprising benzene, toluene, xylenes, ethylbenzene, styrene, higher aromatics (> C8) and non-aromatic components, such as alkanes, cycloalkanes, acetylenics (alkines), diolefins, olefins, paraffins and sulfur containing compounds.
In particular pyrolysis gasoline is a mixture comprising, esp. consisting of, 10 to 50 % by weight non-aromatic components, 15 to 60 % by weight benzene, 10 to 30 % by weight toluene, 0 to 20 % by weight of xylenes and ethylbenzene, 0 to 10 % by weight styrene and 0 to 10 % by weight higher aromatics (> C8).
In particular pyrolysis gasoline is defined as described in Ullmann's Encyclopedia of Industrial Chemistry, Benzene, Vol. 5, pages 245-246, 2012 Wiley-VCH Verlag GmbH & Co. KGaA, Weinheim and Process Economics Program Report 182B, Aromatic Processes, by Abe Gelbein, page 4-9 (114), (December 2008).

In a further embodiment, the hydrocarbon steamcracker plant is an ethane steamcracker plant, a LPG (= Liquefied Petroleum Gas) steamcracker plant, a light naphtha steamcracker plant, a fullrange naphta steamcracker plant or a heavy naphtha steamcracker plant.

In a further embodiment, the aromatic hydrocarbons separation unit of the catalytic reforming plant of the third step is in addition fed with an aromatic hydrocarbons containing fraction from the catalytic reforming plant (also called reformate).

All values regarding pressure (bar) refer to the absolute pressure.
The unit 'barg' means bar overpressure (i.e. bar over 1 bar).

### Examples

Simulation example of feeding a pygas unit partially with an ethane coupling aromatic hydrocarbons stream

The pygas unit fed with pyrolysis gasoline only and fed partially with pyrolysis gasoline and partially with ethane coupling BTX product was simulated using commercial simulation software Aspen Plus®. The pygas unit consisting of the following stages:
1. Hydrogenation Step for acetylenes and dienes
2. C5 splitter
3. Hydrogenation step for sulfur containing components
4. H2S Stripping Column
5. TX Separation Column
6. Benzene Extractive distillation columns

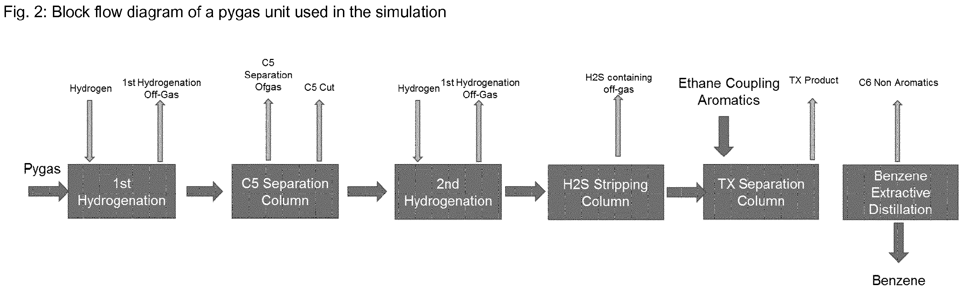

A block flow diagram of the simulated pygas unit is given on Fig. 2.
The simulation was done having a fixed stream of benzene product with 3 cases:
1. Case 1 - only pyrolysis gasoline feed from a steamcracker (pygas feed)
2. Case 2 - 25 % of benzene produced from ethane coupling BTX feed
3. Case 3 - 50 % of benzene produced from ethane coupling BTX feed

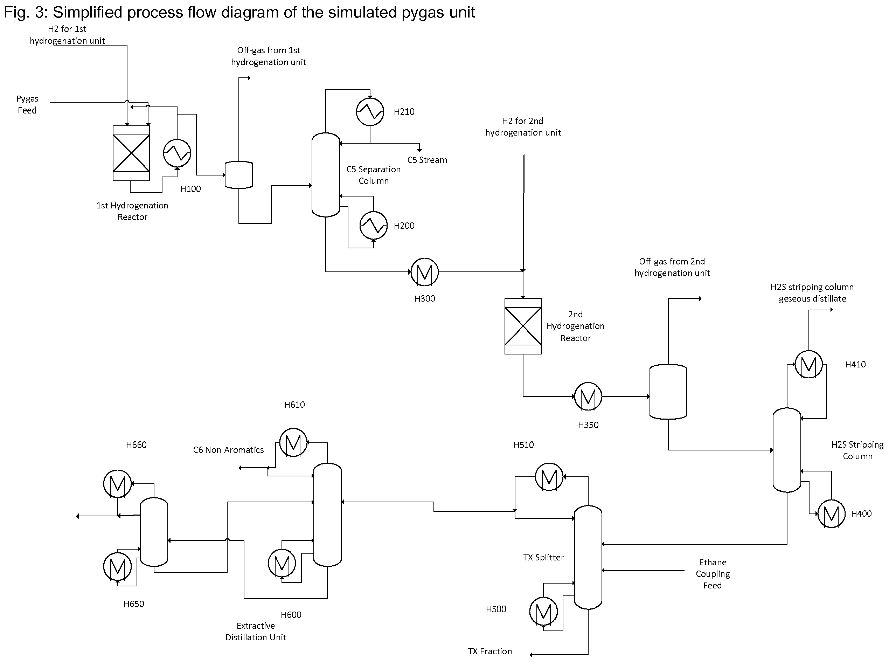

The results of the simulation show mass balance based advantage and energetic advantage of using the ethane coupling aromatic hydrocarbons as co-feed together with pyrolysis gasoline compared with using only pyrolysis gasoline. The results show the advantage of the ethane coupling process as a drop-in unit in the steam cracker and pygas unit system. The advantage lays in a better mass and energy balance.
Table 1 contains the mass balance of the pygas unit for the three above mentioned cases. The mass balance was given in normalized unit - pygas feed for case 1 has the value 100 mass units. Table 2 contains the energy balance, with normalized energy units, of the heat exchangers in the simulated pygas unit. The heat exchangers are showed on a simplified process flow diagram of the pygas unit on Fig. 3.

**Table 1. Mass Balance of the pygas unit for respective cases**

| | Case 1 | Case 2 | Case 3 |
|---|---|---|---|
| Pygas feed to 1^{st} hydrogenation unit [mass unit] | 100,00 | 74,84 | 49,78 |
| H2 for 1^{st} Hydrogenation [mass unit] | 2,81 | 2,38 | 1,93 |
| Off-gas from the 1st hydrogenation unit [mass unit] | 2,96 | 2,60 | 2,22 |
| Product from 1st Hydrogenation / Feed to C5 Separation Column [mass unit] | 99,84 | 74,62 | 49,49 |
| Off-gas from C5 Separation Column [mass unit] | 0,69 | 0,50 | 0,32 |
| C5 Stream [mass unit] | 23,47 | 17,48 | 11,51 |
| C5 Separation Column Bottoms / Feed to 2nd Hydrogenation [mass unit] | 75,69 | 56,64 | 37,65 |
| H2 Needed in 2nd Hydrogenation [mass unit] | 0,51 | 0,38 | 0,25 |
| 2nd Hydrogenation offgas [mass unit] | 0,14 | 0,14 | 0,11 |
| Feed to H2S stripping column [mass unit] | 76,05 | 56,88 | 37,79 |
| H2S stripping column gaseous distillate [mass unit] | 0,33 | 0,23 | 0,14 |
| Pygas feed to TX Separation Column [mass unit] | 75,83 | 69,62 | 63,47 |
| Ethane Coupling Feed to TX Separation Column [mass unit] | 0,00 | 12,96 | 25,82 |
| TX Product [mass unit] | 37,66 | 46,30 | 54,91 |
| Feed to Extractive Distillation [mass unit] | 38,16 | 36,28 | 34,37 |
| C6 Non-Aromatic Hydrocarbons Stream [mass unit] | 7,67 | 5,79 | 3,88 |
| Benzene Product [mass unit] | 30,49 | 30,49 | 30,49 |

**Table 2. Energy Balance of the heat exchangers in the pygas unit**

| | Case 1 | Case 2 | Case 3 |
|---|---|---|---|
| H100 [energy unit] | 100,00 | 77,47 | 52,43 |
| H210 [energy unit] | 100,00 | 76,50 | 52,11 |
| H200 [energy unit] | 100,00 | 74,22 | 48,99 |
| H300 [energy unit] | 100,00 | 58,54 | 27,62 |
| H350 [energy unit] | 100,00 | 76,04 | 49,87 |
| H410 [energy unit] | 100,00 | 66,85 | 39,35 |
| H400 [energy unit] | 100,00 | 58,35 | 21,81 |
| H510 [energy unit] | 100,00 | 92,12 | 81,15 |
| H500 [energy unit] | 100,00 | 93,64 | 85,47 |
| H610 [energy unit] | 100,00 | 75,47 | 50,52 |
| H600 [energy unit] | 100,00 | 71,15 | 49,23 |
| H660 [energy unit] | 100,00 | 77,62 | 61,58 |
| H650 [energy unit] | 100,00 | 81,23 | 78,15 |

The results of the simulation show clear advantage of using of the ethane coupling feed in order to produce benzene. Part of the plant containing the 1st hydrogenation unit, C5 separation column, 2nd hydrogenation unit and stripping column are not needed to process the ethane coupling aromatic hydrocarbons stream. Less hydrogen is needed with increasing amount of ethane coupling feed, less off-gases are produced, as well as more TX (Toluene, Xylenes) cut is produced per amount of benzene and less C5 stream and C6 non-aromatic hydrocarbons stream. The energy amounts for all heat exchangers included in the simulation are lower with increasing ethane coupling feed amount. Therefore, use of the ethane coupling process as a drop is solution in the steam cracker-aromatic-hydrocarbons plant system is of advantage compared with feeding the unit with pyrolysis gasoline only. The composition of the pyrolysis gasoline feedstock and the ethane coupling feedstock used in the simulation is given in Table 3.

**Table 3. Comparison of Ethane Coupling Feed and Pyrolysis Gasoline Feed.**

| Component Name | Concentration in Ethane Coupling Feed [wt. %] | Concentration in Pyrolysis Gasoline Feed [wt. %] |
|---|---|---|
| Benzene | 59,56 | 31,32 |
| Toluene | 31,33 | 16,01 |
| Xylenes | 4,37 | 5,03 |
| Ethylbenzene | 0,62 | 1,45 |
| Napthalene | 4,12 | 0,08 |
| Styrene | 0,00 | 5,82 |
| Lights (C5-) | 0,00 | 22,28 |
| C6NA | 0,00 | 7,34 |
| Other Heavy Boilers | 0,00 | 10,67 |

| | | |
|---|---|---|
| Lights (C5-): C1 to C5 hydrocarbons C6NA: C6 non-aromatics | | |

## Claims

1. A method for producing an aromatic hydrocarbon or a mixture of aromatic hydrocarbons from a low molecular hydrocarbon or a mixture of low molecular hydrocarbons, wherein, in a first step, a low molecular hydrocarbon or a mixture of low molecular hydrocarbons is converted to a mixture of aromatic hydrocarbons by way of a catalytic dehydroaromatization reaction with hydrogen as a byproduct, separating the mixture of aromatic hydrocarbons from hydrogen and non-aromatic hydrocarbons in a second step, and, in a third step, feeding the mixture of aromatic hydrocarbons into an aromatic hydrocarbons separation unit.

2. A method according to claim 1, wherein the aromatic hydrocarbon is benzene, toluene, xylenes, ethylbenzene, styrene, naphthalene or methylnaphthalene and the aromatic hydrocarbons are benzene, toluene, xylenes, ethylbenzene, styrene, naphthalene and/or methylnaphthalene.

3. A method according to claim 1 or 2, wherein the low molecular hydrocarbon is a C2-C4-alkane and the molecular hydrocarbons are C2-C4-alkanes.

4. A method according to one of the preceding claims, wherein in the catalytic dehydroaromatization reaction a zeolite containing Pt is used as catalyst.

5. A method according to one of the preceding claims, wherein in the catalytic dehydroaromatization reaction a ZSM-type zeolite containing Pt and [Ga or Sn] is used as catalyst.

6. A method according to one of the preceding claims, wherein the separation of the mixture of aromatic hydrocarbons from hydrogen and non-aromatic hydrocarbons in the second step is conducted by absorption of the aromatic hydrocarbons in a solvent.

7. A method according to one of the preceding claims, wherein the non-aromatic hydrocarbons in the second step are unconverted low molecular hydrocarbon or unconverted low molecular hydrocarbons, methane and/or olefins.

8. A method according to one of the preceding claims, wherein, in the third step, the mixture of aromatic hydrocarbons is fed into the aromatic hydrocarbons separation unit of a hydrocarbon steamcracker plant or into the aromatic hydrocarbons separation unit of a catalytic reforming plant.

9. A method according to the preceding claim (8), wherein, in the third step, the aromatic hydrocarbons separation unit of the hydrocarbon steamcracker plant is a pyrolysis gasoline unit (pygas unit).

10. A method according to the preceding claim (9), wherein the pygas unit comprises a hydrogenation step for acetylenes and dienes, a C5 splitter, a hydrogenation step for sulfur containing components, a H2S stripping column, a toluene and xylenes separation column and a benzene extractive distillation column.

11. A method according to one of the three preceding claims (8 to 10), wherein the aromatic hydrocarbons separation unit of the hydrocarbon steamcracker plant of the third step is in addition fed with an aromatic hydrocarbons containing fraction from the hydrocarbon steamcracker plant.

12. A method according to claim 8, wherein the aromatic hydrocarbons separation unit of the catalytic reforming plant of the third step is in addition fed with an aromatic hydrocarbons containing fraction from the catalytic reforming plant.

13. A method according to claim 11, wherein the aromatic hydrocarbons containing fraction from the hydrocarbon steamcracker plant is pyrolysis gasoline (pygas).

14. A method according to one of the claims 8 to 11, 13, wherein the hydrocarbon steamcracker plant is a ethane, LPG or naphtha steamcracker plant.

15. A method according to one of the preceding claims, wherein the hydrogen and non-aromatic hydrocarbons separated from the mixture of aromatic hydrocarbons in the second step are fed into a separation unit, resulting in a H2/CH4 - fraction and a low molecular hydrocarbons - fraction (essentially without CH4).

16. A method according to the preceding claim (15), wherein the H2/CH4 - fraction is fed into a further separation unit to separate H2 and CH4.

17. A method according to one of the claims 15 to 16, wherein the low molecular hydrocarbons - fraction (essentially without CH4) is recycled back to the catalytic dehydroaromatization reaction of the first step or used as feed to a hydrocarbon steamcracker plant or a catalytic reforming plant.
